# EUROPEAN PATENT SPECIFICATION

(11) **EP 3 941 443 B1**
(45) Date of publication and mention of the grant of the patent: **13.12.2023**
(21) Application number: 20721145.9
(22) Date of filing: 20.03.2020
(51) Int. Cl.: A61K 9/20, A61K 31/137

(54) **SUSTAINED RELEASE COMPOSITION COMPRISING TAPENTADOL OXALATE AND METHOD OF PREPARATION THEREOF**
ZUSAMMENSETZUNG MIT VERZÖGERTER FREISETZUNG MIT TAPENTADOL-OXALAT UND VERFAHREN ZU IHRER HERSTELLUNG
COMPOSITION À LIBÉRATION PROLONGÉE COMPRENANT DE L'OXALATE DE TAPENTADOL ET SON PROCÉDÉ DE PRÉPARATION

(30) Priority: 22.03.2019 GR 20190100138
(43) Date of publication of application: 26.01.2022
(73) Proprietor: Pharmathen S.A., 15351 Pallini Attikis (GR)
(72) Inventor: KARAVAS, Evangelos, 153 51 Pallini Attikis (GR); KOUTRIS, Efthymios, 153 51 Pallini Attikis (GR); SAMARA, Vasiliki, 153 51 Pallini Attikis (GR); KOUTRI, Ioanna, 153 51 Pallini Attikis (GR); KALASKANI, Anastasia, 153 51 Pallini Attikis (GR); KIZIRIDI, Christina, 153 51 Pallini Attikis (GR); KAKOURIS, Andreas, 153 51 Pallini Attikis (GR)
(86) International application number: PCT/EP2020/025139
(87) International publication number: WO 2020/192969

(56) References cited:
- WO-A1-2017/070566
- WO-A2-2011/124953
- US-A1- 2013 028 970
- US-A1- 2015 272 902
- US-A1- 2015 283 087
- US-A1- 2019 054 024

## Description

### TECHNICAL FIELD OF INVENTION

The present invention relates to a sustained release composition comprising Tapentadol or a pharmaceutically acceptable salt thereof for oral administration for the treatment of severe chronic pain in adults. The composition of the present invention comprises a combination of a hydrophilic and a hydrophobic polymer that is responsible for the sustained release of the active ingredient. Furthermore, the present invention describes a method of preparation of such pharmaceutical composition that is cost effective and will also increase patient compliance.

### BACKGROUND OF THE INVENTION

Tapentadol is well known as a centrally acting opiod analgesic of the benzoid class. It has a dual mode of action as an agonist of the µ-opioid receptor and as a norepinephrine reuptake inhibitor. Compared to morphine it shows 18-times reduced affinity to recombinant µ-opioid receptor suggesting that other pathways may contribute to its analgesic efficacy. Tapentadol was first described in EP 0693475.

Palexia^{®} the immediate release and Palexia SR^{®} the extended release composition are indicated for the management of pain, severe enough to require daily, around-the-clock, long-term opioid treatment and for which alternative treatment options are inadequate. A new indication was added in the USA for the treatment of neuropathic pain associated with diabetic peripheral neuropathy (DPN) in adults severe enough to require daily, around-the-clock, long-term opioid treatment and for which alternative treatment options are inadequate.

In general, the release kinetics of the pharmacologically active ingredients is an important factor. It is well known that depending on how a pharmacologically active ingredient is formulated into a dosage form its release pattern can be modified. Immediate release compositions upon oral administration have the advantage that they lead to a fast release of the pharmacologically active ingredient in the gastrointestinal tract. As a result, a comparatively high dose of the pharmacologically active ingredient is quickly absorbed leading to high plasma levels within a short period of time and resulting in a rapid onset of medicinal action. At the same time, however, a rapid reduction in the medicinal action is observed, because metabolization and/or excretion of the pharmacologically active ingredient cause a decrease of plasma levels. For that reason, formulations providing immediate release of pharmacologically active ingredients typically need to be administered frequently, e.g. up to six times per day. This may cause comparatively high peak plasma, pharmacologically active ingredient concentrations and high fluctuations in plasma concentration of the pharmacologically active ingredient, which in turn could lead to decreased tolerability of the specific active ingredient by the patient. These facts can in turn decrease patient compliance.

In addition, there is an increasing number of patients, especially pediatric patients and geriatric patients, who have difficulties in swallowing monolithic oral dosage forms. This can also affect patient compliance.

Controlled release also known as delayed release, prolonged release, sustained release, extended release and the like, may be based upon various concepts such as coating the pharmaceutical dosage form with a controlled release membrane, embedding the pharmacologically active ingredient in a matrix, binding the pharmacologically active ingredient to an ion-exchange resin, forming a complex of the pharmacologically active ingredient, and the like. In comparison to formulations providing immediate release, formulations providing prolonged release upon oral administration have the advantage that they need to be administered less frequently, typically once daily or twice daily. This can reduce the peak plasma concentration of the pharmacologically active ingredient and any fluctuations that might occur with immediate release compositions which in turn may improve tolerability and increase patient compliance.

WO 2007/093642 claims a modified release form aiming at minimizing the risks of release of the dose associated with the concurrent consumption of alcohol and certain pharmaceutical or dietary forms. It describes an oral dosage form comprising reservoirtype microparticles, with modified release of at least one active principle. The oral form is resistant to the immediate release of the dose of active principle in the presence of alcohol. In particular, the oral form is characterized in that the releasing time of 50% of the active principle, in an alcohol-containing solution is not reduced by more than 3 times compared to the releasing time of 50% of the active principle in an alcohol-free aqueous medium.

WO 2008/033523 claims a pharmaceutical composition comprising a granulate which may include at least one active pharmaceutical ingredient, susceptible to abuse by an individual, mixed with at least two excipients. The composition may also include a coating deposited on the granulate using an alcohol based solvent, said coating being crush resistance.

WO 2010/037854 relates to an oral pharmaceutical form containing microgranules for the sustained release of at least one active ingredient, including a neutral carrier that is insoluble in water or in an alcoholic solution.

WO 2011/124953 describes a once daily controlled release pharmaceutical compositions comprising Tapentadol, wherein preferably the mean Tmax of Tapentadol is reached after 10 hours of administration of the composition. The composition comprises Tapentadol, such that it maintains serum concentration of Tapentadol of at least about 20 ng/ml for at least about 17 hours after oral administration of the composition.

WO 2011/141241 relates to an oral pharmaceutical composition which is resistant to immediate discharge of the dose of active ingredient due to alcohol and which enables a single daily intake.

WO 2012/166474 claims a controlled-release solid dose form ethanol resistant, comprising a film coating composition encapsulating a core, wherein the core comprises an active ingredient; the film coating composition comprises ethylcellulose and guar gum.

However, there still exists a need to develop alternative oral formulations which provide a quick medicinal action while at the same time having the benefits of controlled or modified release formulations.

### SUMMARY OF THE INVENTION

It is, therefore, an object of the present invention to provide a pharmaceutical formulation comprising Tapentadol or a pharmaceutically acceptable salt thereof as set out in the appended claims, that has increased bioavailability and good physicochemical properties. The composition of the present invention is a sustained release composition with a drug release profile of more than 80% after 12 hours of administration.

Another object of the present invention is the preparation of pharmaceutical composition comprising Tapentadol or pharmaceutically acceptable salt thereof together an agent or a combination of agents that will create a matrix core for sustained release of the active ingredient. More particularly the matrix core comprises a hydrophilic polymer in combination with a hydrophobic polymer for sustained release of Tapentadol. More particularly the composition of the present invention comprises Tapentadol oxalate and the ratio of the hydrophilic polymer to the hydrophobic polymer is 1:1.

A further object of the present invention is to provide a sustained release pharmaceutical formulation comprising Tapentadol or a pharmaceutically acceptable salt thereof that has an easy and affordable manufacturing process. The manufacturing process of the present invention is direct compression which comprises dry mixing of the active ingredient with the excipients and compression into tablets.

### DETAILED DESCRIPTION OF THE INVENTION

"Immediate release" means that releases substantially faster than controlled release or delayed release. About 5 to about 20% w/w or more of the total amount of Tapentadol in the composition is released in vitro within about 30 minutes from the beginning of the dissolution, when measured under US Pharmacopoeia (USP) conditions in 0.1N HC1 at 37°C in a type II dissolution apparatus.

As used herein, "sustained release" means that the release of the active ingredient is substantially slower than immediate release. Examples of such sustained release include controlled release, slow release, prolonged release, delayed release, pulsatile release, extended release, timed release etc., which terms are generally known in the art and to the extent they mean a release other than an immediate release. Controlled release may be achieved by various technologies such as reservoir, matrix, osmotic, gastroretention, bioadhesion, complexation, conjugation etc.

One embodiment of the present invention relates to once daily sustained release pharmaceutical compositions comprising Tapentadol, wherein preferably the mean Tmax of tapentadol is reached after 10 hours of administration of the composition.

For the purpose of the present invention Tapentadol includes various forms of Tapentadol such as pharmaceutically acceptable salt(s), hydrate(s), solvate(s), polymorph(s), isomer(s), stereoisomer(s), enantiomer(s), racemate(s), ester(s), prodrug(s), derivative(s), analogou(s), metabolite(s) and complex(s) thereof. More specifically for the present invention the preferred active ingredient is Tapentadol oxalate and it may be present from 1 to 90% w/w of the total composition.

The term "pharmaceutically acceptable salt" means a salt which is acceptable for administration to a patient, such as a mammal (e.g., salts having acceptable mammalian safety for a given dosage regime). Such salts can be derived from pharmaceutically acceptable inorganic or organic bases and from pharmaceutically acceptable inorganic or organic acids.

Composition includes, without limitation, tablets, capsules, caplets, powders, pellets, granules, liquid dispersions, beads, etc. In some aspects, powders, pellets, and granules may be coated with a suitable polymer or a conventional coating material to achieve, for example, greater stability in the gastrointestinal tract, or to achieve the desired rate of release. Moreover, capsules containing a powder, pellets, or granules may be further coated. Tablets may be minitablets, multi-layered tablets, coated or uncoated tablets, tablet in tablet etc. It may also include kits. These compositions can be administered orally.

The term "core" as used herein may be a part of the composition surrounded by at least a part of the coating or layer. The core can be homogenous or have an internal structure comprising powder, particles, granules, pellets, tablets, minitablets, capsules, caplets, or a mixture thereof, comprising active ingredient(s) or carriers/ substrates or a mixture thereof. Core may be prepared by addition of excipients, binder, disintegrant, lubricant and so on, as would be understood by one of ordinary skill in the art. Inert core used herein may be the part of the composition surrounded by at least a part of the coating or layer, which does not comprise active ingredient.

As used herein, "%" refers to the weight percent of a substance as it relates to the overall composition unless otherwise indicated.

The term "comprising", which is synonymous with "including", "containing", or "characterized by" here is defined as being inclusive or open-ended, and does not exclude additional, unrecited elements or method steps, unless the context clearly requires otherwise.

The once daily sustained release pharmaceutical composition comprises Tapentadol and one or more pharmaceutically acceptable excipient(s) which includes release controlling agents, and may optionally contain binders, diluents, lubricants, glidants and plasticizers etc. The amount of excipient employed will depend upon the quantity of active ingredient to be used.

Therefore the main objective of the present invention is the preparation of a sustained release pharmaceutical composition comprising Tapentadol or a pharmaceutically acceptable salt thereof together with an agent or a combination of agents that will create a matrix core for sustained release of the active ingredient.

Agents for sustained release are defined as hydrophilic or hydrophobic agents, which can be polymeric or non-polymeric and which are capable of controlling the rate or release of the active agent(s). The agents for sustained release may be natural, semisynthetic and synthetic agents or mixtures thereof. The agents can be used from about 1 to about 70% of the total composition. The hydrophobic release controlling agents comprises but are not limited to hydrogenated vegetable oil, but other suitable agents include purified grades of beeswax; fatty acids; long chain fatty alcohols, such as cetyl alcohol, myristyl alcohol, and stearyl alcohol; glycerides such as glyceryl esters of fatty acids like glyceryl monostearate, glyceryl distearate, glyceryl esters of hydrogenated castor oil and the like; oils such as mineral oil and the like, or acetylated glycerides; ethyl cellulose, stearic acid , paraffin, carnauba wax, talc; and the stearate salt(s) such as calcium, magnesium, zinc and other materials known to the person skilled in the art.

Natural release controlling agents include but are not limited to proteins (e.g., hydrophilic proteins), such as pectin, zein, modified zein, casein, gelatin, gluten, serum albumin, or collagen, chitosan, oligosaccharides and polysaccharides such as cellulose, dextrans, tamarind seed polysaccharide, gellan, carrageenan, xanthan gum, gum Arabic, guar gum, locust bean gum; hyaluronic acid, polyhyaluronic acid, alginic acid, sodium alginate.

Synthetic release controlling agents are selected from but are not limited to polyamides, polycarbonates, polyalkylenes, polyalkylene glycols such as poly(ethylene glycol), polyalkylene oxides, polyalkylene terephthalates, polyvinyl alcohols (PVA), polyvinylphenol, polyvinyl ethers, polyvinyl esters, polyvinyl halides, polyvinylpyrrolidone (PVP), polyglycolides, polysiloxanes, polyurethanes, polystyrene, polylactides, poly (butyric acid), poly (valeric acid), poly(lactide-co-glycolide), poly (ethyleneterephthalate), poly (lactide-co-caprolactone), polyanhydrides (e.g., poly (adipic anhydride)), polyorthoesters, poly(fumaric acid), poly(maleic acid), polyvinyl acetate, polystyrene; polymers of acrylic and methacrylic esters; carbomer, carbopol; celluloses and cellulose derivatives such as methyl cellulose (MC), ethyl cellulose (EC), hydroxypropyl cellulose (HPC), hydroxypropylmethyl cellulose (HPMC), hydroxybutylmethyl cellulose, sodium carboxymethyl cellulose, cellulose acetate, cellulose propionate, cellulose acetate butyrate, cellulose acetate phthalate, carboxymethyl cellulose, cellulose triacetate, cellulose sulfate sodium salt and blends and copolymers thereof or mixtures thereof.

Representative examples of release controlling agents which swell are selected from, but are not limited to, water-soluble polymers (hydrophilic) such as polyethylene oxide and cellulosic polymer derivatives including hydroxypropyl cellulose, hydroxypropyl methyl cellulose (HPMC), hydroxyethyl cellulose, sodium carboxy methylcellulose, calcium carboxymethyl cellulose, methyl cellulose, as well as noncellulosic such as maltodextrin, polyvinyls, polyvinyl alcohol, polyacrylic acids, alginates, gelatin, natural gums, including guar, lightly crosslinked versions of these polymers, starches, starch graft copolymers and the like. The polymers generally have number average molecular weights over 50,000 grams per mole, such as between 50,000 and 10,000,000 grams per mole. Polymers having molecular weights between 300,000 and 8,000 000 grams per mole are preferred, and those having molecular weights between about 2,000,000 to 8,000,000 grams per mole are especially preferred. Polyethylene oxide having an average molecular weight between about 5,000,000 to 8,000,000 grams per mole is most especially preferred, e.g. Polyox 303 and Polyox 308. Also, especially preferred are methylcellulose type/grade A15C, A4M, A18 M and hydroxypropyl methylcellulose type/grade K4M, K15M, K100M, E4M and F4M (Dow Chemical Company); hydroxyethyl cellulose such as Natrosole HEC; hydroxypropyl cellulose such as Klucel (Grades H, M, G, J, L, E- Aqualon Company); guar such as Supercol^{®} Guar U (Aqualon Company); pectin such as GENU Pectin (Aqualon Company); carrageenan such as GENU Carrageenan (Aqualon Company); poly(methyl vinyl ether/maleic anhydride) such as Gantrez^{®} AN Copolymer (AN- 119, -139, -149, -169, -179, GAF Corporation); polyvinyl alcohol such as Elvanol^{®} 71-30, Elvanol^{®} 85-80, Elvanol^{®} 55- 65, Elvanol^{®} 50-42 and Elvanol^{®} HV (DuPont); sodium carboxymethyl cellulose such as Aqualon cellulose gum grade 7H4; polyacrylic acids such as Carpobol resin grades 934P, 940, 941, 971P, 974P, 980, 981, 1382, 2984, 5984, ETD 2001, ETD 2050, calcium polyacrylic acids such as Noveon^{®} resin grades AA-1, CA-1 and CA-2, and sodium polyacrylic acid (BF Goodrich, Cleveland, Ohio). More preferred is hydroxypropyl methylcellulose and combinations thereof.

The concentration of such hydrophilic polymer in the composition can be in the range of 5 to 40% by weight of the total composition, preferably from about 10 to about 30% by weight of the total composition and most preferably from about 15 to about 20% by weight of the total composition. According to the invention, polyethylene oxide of high molecular weight i.e. above 1,000,000 is used as the hydrophilic polymer.

The term "pH-dependent polymers" refers to the polymers which are relatively insoluble (hydrophobic) and impermeable at the pH of the stomach, but which are more soluble or disintegrable or permeable at the pH of the small intestine and colon. The hydrophobic polymers are selected from, but are not limited to, polyacrylamides, phthalate derivatives such as acid phthalates of carbohydrates, amylose acetate phthalate, cellulose acetate phthalate, other cellulose ester phthalates, cellulose ether phthalates, hydroxypropylcellulose phthalate, hydroxypropylethylcellulose phthalate, hydroxypropylmethylcellulose phthalate, methylcellulose phthalate, polyvinyl acetate phthalate, polyvinyl acetate hydrogen phthalate, sodium cellulose acetate phthalate, starch acid phthalate, succinates such as hydroxypropylethylcellulose acetyl succinate, cellulose acetate trimellitate, styrene-maleic acid dibutyl phthalate copolymer, styrene-maleic acid polyvinylacetate phthalate copolymer, styrene and maleic acid copolymers, polyacrylic add derivatives such as acrylic acid and acrylic ester copolymers, polymethacrylic acid and esters thereof, poly acrylic methacrylic add copolymers, shellac, and vinyl acetate and crotonic add copolymers and combinations thereof.

The hydrophobic polymers for delayed controlled release of active ingredients are the release controlling agents which delay the release of the active ingredients from the composition. Non limiting examples of this type of polymers include cellulosic derivatives including ethylcellulose, hydroxypropyl cellulose, hydroxypropyl methyl cellulose, hydroxyethyl cellulose, sodium carboxy methylcellulose, calcium carboxymethyl cellulose, methyl cellulose, as well as noncellulosics such as maltodextrin, polyvinyls, polyvinyl alcohol, and co-polymers of acrylic and methacrylic acid esters (Eudragit^{®} RS or RL).

Kollidon products are also hydrophobic can also be used and consist of soluble and insoluble grades of polyvinylpyrrolidone (PVP) of various molecular weights and particle sizes. The polymers are used as dry binders, film-formers, stabilizers in suspensions, dispersants for pigments, enzyme stabilizers, and for improvement of bioavailability.

Kollidon SR is a polyvinyl acetate and povidone based matrix retarding agent. It is particularly suitable for the manufacture of sustained release matrix tablets by direct compression. Polyvinyl acetate is a plastic material that produces a coherent matrix even under low compression forces. The water soluble povidone leaches out upon contact with water or gastric fluids forming pores through which the active ingredient slowly diffuses outwards. The blend of polyvinyl acetate and povidone (K 30) that is used in accordence with the invention is in the ratio 8:2.

The concentration of such hydrophobic polymer in the composition can be in the range of 5 to 40% by weight of the total composition, preferably from about 10 to about 30% by weight of the total composition and most preferably from about 15 to about 20% by weight of the total composition.

Delaying release of active ingredient can also be achieved by coating the powder, granules, pellets, tablets, minitablets, capsules of the active ingredients or a mixture of active ingredient and excipients with the release delaying agents. Delaying release of active ingredient can also be achieved by coating inert core with a matrix of active ingredients and the release delaying agents. The active ingredients and release delaying agents are dissolved or dispersed in a suitable solvent and then coated the inert core by different techniques known in art.

Non limiting examples of binders include starches such as potato starch, wheat starch, corn starch, celluloses such as hydroxypropyl cellulose (HPC), hydroxyethyl cellulose, hydroxypropylmethyl cellulose (HPMC), ethyl cellulose, sodium carboxy methyl cellulose; natural gums like acacia, alginic acid, guar gum; liquid glucose, dextrin, povidone, syrup, polyethylene oxide, polyvinyl pyrrolidone, poly-N-vinyl amide, polyethylene glycol, gelatin, poly propylene glycol, tragacanth, combinations thereof and other materials known to one of ordinary skill in the art and mixtures thereof. The preferred binder of the present invention is polyvinyl pyrrolidone and the amount may vary within the range of from about 0.1% to about 10% by weight of the total composition and most preferably it is 2% by weight of the total composition.

Fillers or diluents include, but are not limited to dextrates, dextrin, dextrose, fructose, lactitol, mannitol, sucrose, starch, lactose, xylitol, sorbitol, talc, microcrystalline cellulose, calcium carbonate, calcium phosphate dibasic or tribasic, calcium sulphate or mixtures thereof. The preferred filler or diluent of the present invention is microcrystalline cellulose and the amount may vary within the range of from about 10% to about 60% by weight of the total composition and most preferably it is 24% by weight of the total composition.

Lubricants may be selected from, but are not limited to, those conventionally known in the art such as magnesium, aluminium or calcium or zinc stearate, polyethylene glycol, glyceryl behenate, mineral oil, sodium stearyl fumarate, stearic acid, hydrogenated vegetable oil and talc or mixtures thereof. The preferred lubricant of the present invention is magnesium stearate and the amount may vary within the range of from about 0.1% to about 2.0% by weight of the total composition and most preferably it is 1.0% by weight of the total composition.

Glidants include, but are not limited to, silicon dioxide, fumed silica, magnesium trisilicate, powdered cellulose, starch, talc and tribasic calcium phosphate, calcium silicate, magnesium silicate, colloidal silicon dioxide, silicon hydrogel or mixtures thereof. The prefer glidant of the present invention is Aerosil^{®} and the amount may vary within the range of from about 0.1% to about 10% by weight of the total composition and most preferably it is 2% by weight of the total composition.

The controlled release pharmaceutical composition(s) may optionally contain a surfaceactive agent or solubilizing agents. Solubilizing agents help to solubilize the active ingredient either in composition or in-situ at the site of absorption or action. Solubilizing agents include but are not limited to surfactants, cyclodextrin and its derivatives, lipophilic substances or any combination thereof. Non-limiting examples of surfactants include water soluble or water dispersible nonionic, semi-polar nonionic, anionic, cationic, amphoteric, or zwitterionic surface active agents or any combination thereof.

The preferred surface active agents include, but are not limited to, copolymers composed of a central hydrophobic chain of polyoxypropylene (poly (propylene oxide)) and polyoxyethylene (poly (ethylene oxide)) that is well known as poloxamer. However, other agents may also be employed such as dioctyl sodium sulfosuccinate (DSS), triethanolamine, sodium lauryl sulphate (SLS), polyoxyethylene sorbitan and poloxalkol derivatives, quaternary ammonium salts or other pharmaceutically acceptable surface active agents known to the person skilled in the art. Other solubilizing agents include but not necessarily limited to vitamin E and its derivatives; monohydric alcohol esters such as trialkyl citrates, lactones and lower alcohol fatty acid esters; nitrogen-containing solvents; phospholipids; glycerol acetates such as acetin, diacetin and triacetin; glycerol fatty acid esters such as mono-, di- and triglycerides and acetylated mono- and di-glycerides; propylene glycol esters; ethylene glycol esters and combinations thereof.

The once daily controlled release pharmaceutical composition may be manufactured by various methods such as by dry granulation, wet granulation, melt granulation, direct compression, double compression, extrusion spheronization and layering. The process may be carried out under ambient conditions of temperature and humidity.

The once daily controlled release pharmaceutical composition may optionally have one or more non-functional coatings such as film coating or sugar coating, which has no or negligible impact on release of active ingredient form the composition. The controlled release pharmaceutical composition may further have one or more functional coating such as bioadhesive coating, diffusion coatings, non-permeable coating and semipermeable coating, which modify the release of active ingredients from the composition.

The coating layers may comprise one or more excipients selected from the group comprising coating agents, opacifiers, taste-masking agents, fillers, polishing agents, colouring agents, antitacking agents, pore forming agents and the like. Coating agents include, but are not limited to, polysaccharides such as maltodextrin, alkyl celluloses such as methyl or ethyl cellulose, hydroxyalkylcelluloses (e.g. hydroxypropylcellulose or hydroxypropylmethylcelluloses); polyvinylpyrrolidone, acacia, com, sucrose, gelatin, shellac, cellulose acetate pthalate, lipids, synthetic resins, acrylic polymers, opadry, polyvinyl alcohol (PVA), copolymers of vinylpyrrolidone and vinyl acetate and polymers based on methacrylic acid such as those marketed under the brand name of Eudragit^{®}.

The coating may be applied from aqueous or non-aqueous systems or combinations of aqueous and non-aqueous systems, as appropriate. Excipients can be included along with the film formers to obtain satisfactory films. These excipients can include plasticizers such as dibutyl phthalate, triethyl citrate, polyethylene glycol (PEG) and the like, antitacking agents such as talc, stearic acid, magnesium stearate and colloidal silicon dioxide and the like, surfactants such as polysorbates and sodium lauryl sulphate, fillers such as talc, precipitated calcium carbonate, polishing agents such as beeswax, carnauba wax, synthetic chlorinated wax and opacifying agents such as titanium dioxide and the like. All these excipients can be used at levels well known to the persons skilled in the art. The coating can be done by any method known in the art.

Various coating methods known in the art are pan coating, spray coating, compression coating, dip coating etc.

In another embodiment the once daily controlled release pharmaceutical comprising tapentadol or combinations with other active ingredients can be used for the treatment of pain. The pain can be acute or chronic and may vary from mild to moderate to severe. Treatment of pain includes but not limited to treatment of, chronic low back pain, acute low back pain, acute pain after abdominal hysterectomy, acute pain from bunionectomy, postoperative pain following bunionectomy surgery, acute pain after hip replacement surgery, moderate to severe chronic pain due to osteoarthritis of the knee chronic tumor related pain, chronic malignant tumor related pain, cancer pain, chronic malignant tumor- related cancer pain, awaiting joint replacement surgery, post-surgical pain in children and adolescents, acute pain from vertebral compression fracture associated with osteoporosis, treatment of acute post-operative pain following elective arthroscopic shoulder surgery, treatment of moderate to severe pain in subjects with knee osteoarthritis, treatment in patients with end-stage joint disease, painful diabetic peripheral neuropathy, moderate to severe pain due to chronic, painful diabetic peripheral neuropathy (DPN), treatment of chronic tumor related pain, postherpetic neuralgia. The once daily controlled release pharmaceutical comprising tapentadol or combinations with other active ingredients can also be used for treatment of other diseases known in art.

As previously stated, the main objective of the present invention is the preparation of a sustained release pharmaceutical composition comprising Tapentadol oxalate together with a combination of agents, more preferably a hydrophilic polymer and a hydrophobic polymer in a ratio 1:1 that will create a matrix core for sustained release of the active ingredient. The matrix core can be optionally coated. The manufacturing process of the present invention is a simple procedure of direct compression, which is cost effective.

### EXAMPLES

### Example 1

The inventors first attempted a composition comprising a matrix core for sustained release comprising a hydrophilic polymer and a hydrophobic polymer that in combination act as a gelling agent.

Eudragit RS was used as a non-swellable, insoluble component in tablet matrix with low permeability that enables time controlled release of the active ingredient by pH-independent swelling. Eudragit RS and Kollidon SR were used alternatively as hydrophobic polymers and Polyethylene oxide of high molecular weight (Polyox WSR303) as hydrophilic/gelling polymer as shown below (Table 1: Formulations 1 and 2). The manufacturing process that was applied was direct compression, which comprises dry mixing followed by compression due to the fact that the swellable/gelling polymer cannot be easily processed in the presence of water.

**Table 1: Qualitative and quantitative composition of Formulations 1 and 2**

| | **Formulation 1 w/w %** | **Formulation 2 w/w %** |
|---|---|---|
| **Internal phase** | | |
| Tapentadol oxalate | 39,00 | 39,00 |
| Polyox WSR303 | 38,00 | 38,00 |
| Eudragit RS | 5,00 | - |
| Kollidon SR | - | 5,00 |
| Microcrystalline cellulose | 13,00 | 13,00 |
| Povidone (PVP) | 2,00 | 2,00 |

| **External phase** | | |
|---|---|---|
| Aerosil | 2,00 | 2,00 |
| Magnesium stearate | 1,00 | 1, 00 |
| **Total (uncoated)** | 100,00 | 100,00 |

Both formulation trials resulted in similar drug release however Formulation 2 with Kollidon SR exhibited slightly better physical properties and was thus selected for further trials.

### Example 2

In order to further optimize formulation 2, the inventors different formulations adjusting the ratio between the two polymers. Formulation 3 A-E were prepared with the same manufacturing process as before and are shown in Table 2.

**Table 2: Qualitative and quantitative composition of Formulations 3 A-E**

| **Formulation** | **3A** | **3B** | **3C** | **3D** | **3E** |
|---|---|---|---|---|---|
| **Internal phase** | % | % | % | % | % |
| Tapentadol oxalate | 39,00 | 39,00 | 39,00 | 39,00 | 39,00 |
| Polyox WSR303 | 30,00 | 10,00 | 10,00 | 20,00 | 30,00 |
| Kollidon SR | 10,00 | 30,00 | 10,00 | 20,00 | 30,00 |
| Microcrystalline cellulose | 16,00 | 16,00 | 36,00 | 16,00 | - |
| Povidone (PVP) | 2,00 | 2,00 | 2,00 | 2,00 | - |

| **External phase** | | | | | |
|---|---|---|---|---|---|
| Aerosil | 2,00 | 2,00 | 2,00 | 2,00 | - |
| Magnesium stearate | 1,00 | 1,00 | 1,00 | 1,00 | 1,00 |
| **Total (uncoated)** | 100,00 | 100,00 | 100,00 | 100,00 | 100,00 |

The formulations were tested for their dissolution profiles in USP II apparatus, pH 6.8, 100rpm and data of 1h, 4h and 12h. The % drug release was used as responses to determine the proper ratio of hydrophilic and hydrophobic polymer. The results showed both polymers affect significantly the drug release. Surprisingly, when the amount of one polymer is higher compared to the other, the drug release is very fast. Drug release closer to the target values was achieved when equal amounts of the polymers to middle level (20%) were used in the formulation.

### Example 3

As a next step optimization of the polymers amount was performed preparing formulation trials 4 and 5 with equal amounts of Polyox and Kollidon SR in which their combined amount was reduced to 16% and 18% respectively. Formulations trials 4 and 5 were developed with the same manufacturing technique as before and are shown in Table 3.

**Table 3: Qualitative and quantitative composition of Formulations 4 and 5**

| **Ingredients** | **Formulation 4** | **Formulation 5** |
|---|---|---|
| **Internal phase** | **%** | **%** |
| Tapentadol oxalate | 39,00 | 39,00 |
| Polyox WSR303 | 16,00 | 18,00 |
| Kollidon SR | 16,00 | 18,00 |
| Microcrystalline cellulose | 24,00 | 20,00 |
| Povidone (PVP) | 2,00 | 2,00 |

| **External phase** | | |
|---|---|---|
| Aerosil | 2,00 | 2,00 |
| Magnesium stearate | 1,00 | 1,00 |
| **Total for uncoated** | 100,00 | 100,00 |

The formulations were tested for their dissolution profiles in USP II apparatus, pH 6.8, 100 rpm for 12 hours and % drug release is shown in Table 4.

**Table 4: %drug release for Formulation 4 and 5**

| | **Formulation 4** | **Formulation 5** |
|---|---|---|
| **Time (h)** | **% release** | **% release** |
| 1 | 21,58 | 23,04 |
| 2 | 34,07 | 33,84 |
| 4 | 53,11 | 50,28 |
| 6 | 67,32 | 62,77 |
| 8 | 81,77 | 73,16 |
| 12 | 97,08 | 92,40 |

Formulation 4 showed a Hardness of 205N and Carr's index of 25.5% and Formulation 5 showed a Hardness of 190N and Carr's index of 26.0%. According to the dissolution profiles shown in Table 4 and in combination with the other measurements, Formulation 4 shows a dissolution profile closer to the target specifications.

### Example 4

Subsequently the effect of the tablet filler was evaluated. Microcrystalline cellulose that was used in previous trials provided very good compressibility and tablets with high hardness. However it is insoluble in water and may affect the dissolution profile. Therefore Formulation 6 shown in Table 5 below was prepared with lactose monohydrate which is a water-soluble diluent and can enhance drug release. The same manufacturing process as before was used.

**Table 5: Qualitative and quantitative composition of Formulations 6**

| **Ingredients** | **Formulation 6** |
|---|---|
| **Internal phase** | % |
| Tapentadol oxalate | 39,00 |
| Polyox WSR303 | 16,00 |
| Kollidon SR | 16,00 |
| Lactose monohydrate | 24,00 |
| Povidone (PVP) | 2,00 |

| **External phase** | |
|---|---|
| Aerosil | 2,00 |
| Magnesium stearate | 1,00 |
| **Total for uncoated** | 100,00 |

**Table 6: % drug release for Formulation 6**

| | **Formulation 6** |
|---|---|
| **Time (h)** | **% release** |
| 1 | 23,14 |
| 2 | 36,14 |
| 4 | 55,19 |
| 6 | 69,44 |
| 8 | 78,07 |
| 12 | 91,80 |

Formulation 6 showed a Hardness of 147N and Carr's index of 29.0%. According to the results even though the drug release was enhanced with the use of lactose however lower compressibility and lower hardness were achieved.

### Example 5

In order to combine the high solubility of lactose with the good compaction properties of Microcrystalline cellulose Formulation trial 7 was prepared using Microcelac 100 (75% lactose- 25% microcrystalline cellulose) as diluent and the same manufacturing process as before.

**Table 7: Qualitative and quantitative composition of Formulations 7**

| **Ingredients** | **Formulation 7** |
|---|---|
| **Internal phase** | % |
| Tapentadol oxalate | 39,00 |
| Polyox WSR303 | 16,00 |
| Kollidon SR | 16,00 |
| Microcelac 100 | 24,00 |
| Povidone (PVP) | 2,00 |

| **External phase** | |
|---|---|
| Aerosil | 2,00 |
| Magnesium stearate | 1,00 |
| **Total (uncoated)** | 100,00 |

**Table 8: %drug release for Formulation 7**

| | **Formulation 7** |
|---|---|
| **Time (h)** | **% release** |
| 1 | 22,90 |
| 2 | 35,53 |
| 4 | 54,31 |
| 6 | 68,75 |
| 8 | 80,21 |
| 12 | 95,74 |

Formulation 7 showed a Hardness of 186N and Carr's index of 25.0%. Using Microcelac 100 resulted in better physical properties than using lactose and thus it was included in the formula as the proper diluent.

### Example 6

Finally, formulation trial 8 was prepared using Copovidone (Kollidon VA64) as binder instead of PVP to study its effect on the physical properties and dissolution profile.

**Table 9: Qualitative and quantitative composition of Formulations 8**

| **Ingredients** | **Formulation 8** |
|---|---|
| **Internal phase** | **%** |
| Tapentadol oxalate | 39,00 |
| Polyox WSR303 | 16,00 |
| Kollidon SR | 16,00 |
| Microcelac 100 | 24,00 |
| Copovidone (Kollidon VA64) | 2,00 |

| **External phase** | |
|---|---|
| Aerosil | 2,00 |
| Magnesium stearate | 1,00 |
| **total for uncoated** | 100,00 |

Formulation 8 showed a Hardness of 203N and Carr's index of 24.0% Since the physical characteristics of Formulation trial 8 were the desirable and in order to conclude to the final formula the dissolution profile was compared with the reference product as presented below in table 10.

**Table 10: %drug release for Formulation 8 compared to reference product**

| | **Reference product** | **Formulation 8** |
|---|---|---|
| **Time (h)** | **% release** | **% release** |
| 1 | 27,93 | 25,8 |
| 2 | 40,97 | 39,3 |
| 4 | 59,91 | 58,3 |
| 6 | 73,33 | 70,3 |
| 8 | 83,10 | 80,3 |
| 12 | 94,11 | 92,5 |
| **F2** | | **79,9** |

According to the dissolution results similar profile with the reference product was achieved with Formulation 8 so this was selected as the final formula. In order to test the chemical stability of the API in the final product tablets of Formulation trial 8 were stored under normal and accelerated conditions for 6 months.

The stability data are presented in the following table 11. The results presented confirm that the tablets remain stable after 6 months storage under normal and accelerated conditions.

**Table 11: Stability studies for Formulation 8**

| | **STABILITY DATA OF TRIAL 8** | | |
|---|---|---|---|
| **SPECIFICATION** | **Zero time** | **6months 25°C/60% RH** | **6months 40°C/ 75%RH** |
| **Total impurities NMT 2.0%** | 0.15% | 0.21% | 0.22% |

## Claims

1. A sustained release pharmaceutical composition for oral administration comprising Tapentadol or pharmaceutically acceptable salt thereof in a matrix core comprising a hydrophilic and a hydrophobic polymer at polymer ratio 1: 1, wherein the hydrophilic polymer is polyethylene oxide of high molecular weight and the hydrophobic polymer is a blend of polyvinyl acetate and povidone in the ratio 8:2.

2. A sustained release pharmaceutical composition according to claim 1, wherein Tapentadol oxalate is the active pharmaceutical ingredient.

3. A sustained release pharmaceutical composition according to claim 1, wherein it further comprises lactose, microcrystalline cellulose and copovidone as pharmaceutically acceptable excipients.

4. A sustained release pharmaceutical composition according to claim 1, wherein it further comprises a glidant and/or a lubricant.

5. A process for the manufacture of a sustained release pharmaceutical composition for oral administration comprising Tapentadol or pharmaceutically acceptable salt thereof in a matrix core comprising a hydrophilic and a hydrophobic polymer, at polymer ratio 1:1, wherein the hydrophilic polymer is polyethylene oxide of high molecular weight and the hydrophobic polymer is a blend of polyvinyl acetate and povidone in the ratio 8:2, wherein said process comprises the steps of:
- Blending the total amount of Tapentadol or pharmaceutically acceptable salt thereof with a hydrophilic and a hydrophobic polymer until complete homogeneity;
- Adding a lubricant and or a glidant to the above mixture and mixing until complete homogeneity;
- Compressing into tablets
- Optionally, coating the tablets with a water soluble coating agent.

6. The process according to claim 5, wherein the mixture of Tapentadol or pharmaceutically acceptable salt thereof with a hydrophilic and a hydrophobic polymer further comprises lactose, microcrystalline cellulose and copovidone as pharmaceutically acceptable excipients

## Patentansprüche

1. Pharmazeutische Zusammensetzung mit verzögerter Wirkstofffreisetzung zur oralen Verabreichung, welche Tapentadol oder ein pharmazeutisch verwendbares Salz davon in einem Matrixkern umfasst, der ein hydrophiles und ein hydrophobes Polymer im Polymerverhältnis 1:1 umfasst, wobei das hydrophile Polymer Polyethylenoxid mit hohem Molekulargewicht ist und das hydrophobe Polymer eine Mischung aus Polyvinylacetat und Povidon im Verhältnis 8:2 ist.

2. Pharmazeutische Zusammensetzung mit verzögerter Wirkstofffreisetzung nach Anspruch 1, **dadurch gekennzeichnet, dass** der pharmazeutische Wirkstoff Tapentadoloxalat ist.

3. Pharmazeutische Zusammensetzung mit verzögerter Freisetzung nach Anspruch 1, **dadurch gekennzeichnet, dass** sie weiterhin Lactose, mikrokristalline Cellulose und Copovidone als pharmazeutisch verwendbare Hilfsstoffe umfasst.

4. Pharmazeutische Zusammensetzung mit verzögerter Freisetzung nach Anspruch 1, **dadurch gekennzeichnet, dass** sie ferner ein Gleitmittel und/oder ein Schmiermittel umfasst.

5. Verfahren für die Vorbereitung einer pharmazeutischen Zusammensetzung mit verzögerter Wirkstofffreisetzung zur oralen Verabreichung, welche Tapentadol oder ein pharmazeutisch verwendbares Salz davon in einem Matrixkern umfasst, der ein hydrophiles und ein hydrophobes Polymer im Polymerverhältnis 1:1 umfasst, wobei das hydrophile Polymer Polyethylenoxid mit hohem Molekulargewicht ist und das hydrophobe Polymer eine Mischung aus Polyvinylacetat und Povidon im Verhältnis 8:2 ist, wobei das o.g. Verfahren die nachstehenden Schritte umfasst:
- Die Gesamtmenge von Tapentadol oder vom pharmazeutisch verwendbaren Salz davon mit einem hydrophilen und einem hydrophoben Polymer bis zur vollständigen Homogenität mischen;
- Ein Gleitmittel und/oder Schmiermittel in die obere Mischung hinzufügen und rühren bis die Mischung einheitlich wird;
- Die daraus resultierende Mischung tablettieren.
- Optional die Tabletten mit einem wasserlöslichen Film überziehen.

6. Verfahren nach Anspruch 5, **dadurch gekennzeichnet, dass** die Mischung Tapentadol oder ein pharmazeutisch verwendbares Salz davon, ein hydrophiles und ein hydrophobes Polymer sowie Lactose, mikrokristalline Cellulose und Copovidon als pharmazeutisch verwendbare Hilfsstoffe umfasst.

## Revendications

1. Composition pharmaceutique à libération prolongée pour administration orale comprenant du tapentadol ou un sel pharmaceutiquement acceptable de celui-ci dans un noyau de matrice comprenant un polymère hydrophile et un polymère hydrophobe à un rapport de polymère de 1: 1, dans laquelle le polymère hydrophile est un oxyde de polyéthylène de poids moléculaire élevé et le polymère hydrophobe est un mélange d'acétate de polyvinyle et de povidone dans le rapport de 8:2.

2. Composition pharmaceutique à libération prolongée selon la 1^{ère} revendication, dans laquelle l'oxalate de tapentadol est l'ingrédient pharmaceutique actif.

3. Composition pharmaceutique à libération prolongée selon la 1^{ère} revendication, qui comprend en outre du lactose, de la cellulose microcristalline et de la copovidone en tant qu'excipients pharmaceutiquement acceptables.

4. Composition pharmaceutique à libération prolongée selon la 1^{ère} revendication, qui comprend en outre un glissant et/ou un lubrifiant.

5. Procédé de fabrication d'une composition pharmaceutique à libération prolongée pour administration orale comprenant du tapentadol ou un sel pharmaceutiquement acceptable de celui-ci dans une matrice comprenant un polymère hydrophile et un polymère hydrophobe, dans un rapport de polymère de 1:1, dans lequel le polymère hydrophile est un oxyde de polyéthylène de poids moléculaire élevé et le polymère hydrophobe est un mélange d'acétate de polyvinyle et de povidone dans le rapport de 8:2, dans lequel ledit procédé comprend les étapes consistant :
- Au mélange de la quantité totale de tapentadol ou de son sel pharmaceutiquement acceptable avec un polymère hydrophile et un polymère hydrophobe jusqu'à homogénéité complète ;
- À l'ajout d'un lubrifiant et/ou un glissant au mélange ci-dessus et mélanger jusqu'à homogénéité complète ;
- Compression en forme de comprimés
- Enrobage éventuel des comprimés avec un agent d'enrobage hydrosoluble.

6. Procédé selon l'affirmation 5, dans lequel le mélange de Tapentadol ou d'un sel pharmaceutiquement acceptable de celui-ci avec un polymère hydrophile et un polymère hydrophobe comprend en outre du lactose, de la cellulose microcristalline et de la copovidone comme excipients pharmaceutiquement acceptables
